# DEMANDE DE BREVET EUROPEEN

(11) **EP 2 045 331 A1**
(43) Date de publication de la demande: **08.04.2009**
(21) Numéro de dépôt: 07301425.0
(22) Date de dépôt: 02.10.2007
(51) Int. Cl.: C12N 15/70, A61K 35/74, A61K 48/00

(54) **Souches E. COLI attenuées invasives et leurs applications comme vecteur intracellulaire de molécule thérapeutique**

(71) Demandeur: INSTITUT PASTEUR, 75015 Paris (FR)
(72) Inventeur: Grillot-Courvalin, Catherine, Liliane, Andrée, 75015, PARIS (FR); Goussard, Sylvie, Dominique, 75015, PARIS (FR); Courvalin, Patrice, Marie, Denis, Paul, 75015, PARIS (FR)
(74) Mandataire: Warcoin, Jacques

(57) **Abrégé**

La présente invention concerne un système vectoriel capable de délivrer dans des cellules cibles eucaryotes un acide nucléique d'intérêt, ce système vectoriel comprenant une bactérie *Escherichia coli* (*E. coli*) recombinante non pathogène et non réplicative ayant intégré de façon ciblée et sans marqueur antibiotique, dans son chromosome un ou plusieurs gènes conférant cette bactérie *E. coli* la capacité de pénétrer dans le cytoplasme de ces cellules cibles eucaryotes et de lyser la vacuole de pénétration. La présente invention comprend également l'utilisation de telles bactéries *E. coli* pour la production de compositions thérapeutiques et leur délivrance sans étape de purification destinées à la prévention ou au traitement de maladie par vaccination ou par thérapie génique.

SEQUENCE LISTING

<110> Institut Pasteur

<120> Souches E. coli atténuées invasives et leurs applications
      comme vecteur intracellulaire de molécule thérapeutique

<130> D25834

<160> 12

<170> PatentIn version 3.3


<210> 1
<211> 4398
<212> DNA
<213> Artificial sequence

<220>
<223> Séquence « Delta dapB oméga hly » de BM4570

<400> 1
cgctcgccca aggggccgct gtgccactgg tacgcgcacc gcgtgatgcg gcatctgtgc      60
acggcgaatc gggaatggct ggctacgact ttgttgagca caaccgaaag ccgctcggga     120
taccggcgtt tctggcgatt cgggatgccc tgatgcgtgc accagagcct gttaccctgg     180
tggccatcgg cccgttaacc aatattgcgc tgttactttc acaatgcccg gaatgcaagc     240
cgtatattcg ccgtctggtg atcatgggtg gttctgccgg acgcggcaac tgtacgccaa     300
acgccgagtt taatattgct gccgatccag aagctgctgc ctgtgtcttc cgcagtggta     360
ttgaaatcgt catgtgcggt ttggatgtca ccaatcaggc aatattaact cctgactatc     420
tctctacact gccgcagtta aaccgtaccg ggaaaatgct tcacgccctg tttagccact     480
accgtagcgg cagtatgcaa agcggcttgc gaatgcacga tctctgcgcc atcgcctggc     540
tggtgcgccc ggacctgttc actctcaaac cctgttttgt ggcagtggaa actcagggcg     600
aatttacctc aggcacgacg gtggttgata tcgacggttg cctgggcaag ccagccaatg     660
tacaggtggc attggatctg gatgtgaaag gcttccagca gtgggtggct gaggtgctgg     720
ctctggcgtc gtaacctgtc acatgttatt ggcatgcagt cattcatcga ctcatgcctt     780
tcactgatat ccctccctgt ttatcattaa tttctaatta tcagcgtttt tggctggcgg     840
cgtagcgatg cgctggttac tctgaaaacg gtctatgcaa attaacaaaa gagaatagct     900
atgcatgatg caaacatccg cgttgccatc gcgggagccg gggggcgtat gggccgccag     960
ttgattcagg cggcgctggc attagagggc gtgcagttgg gcgctgcgct ggagcgtgaa    1020
ggatcttctt tactgggcag cgacgccggt gagctggccg gagccgggaa aacaggcgtt    1080
accgtgcaaa gcagcctcga tgcggtaaaa gatgattttg atgtgtttat cgattttacc    1140
cgtccggaag gtacgctgaa ccatctcgct tttgtcgccc agcatggcaa agggatggtg    1200
atcggcacta cggggtttga cgaagccggt aaacaagcaa ttcgtgacgc cgctgccgat    1260
attgcgattg tctttgctgc caattttagc gttggcgtta acgtcatgct taagctgctg    1320
gagaaagcag ccaaagtgat gggtgactac actagataac tgaggatctg aagttcctat    1380
actttctaga gaataggaac ttcggaatag gaacttcaga tcctcaaatg tagcacctga    1440
agtcagcccc atacgatata agttgtaatt ctcatgtttg acagcttatc atcgataagc    1500
tttaatgcgg tagtttatca cagttaaatt gctaacgcag tcaggcaccg tgtatgaaat    1560
ctaacaatgc gctcatcgtc atcctcggca ccgtcaccct ggatgctgta ggcataggct    1620
tggttatgcc ggtactgccg ggcctcttgc gggatatcct ctagaaataa ttttgtttaa    1680
ctttaagaag gagatataca tatgaaggat gcatctgcat tcaataaaga aaattcaatt    1740
tcatccatgg caccaccagc atctccgcct gcaagtccta agacgccaat cgaaaagaaa    1800
cacgcggatg aaatcgataa gtatatacaa ggattggatt acaataaaaa caatgtatta    1860
gtataccacg gagatgcagt gacaaatgtg ccgccaagaa aaggttacaa agatggaaat    1920
gaatatattg ttgtggagaa aaagaagaaa tccatcaatc aaaataatgc agacattcaa    1980
gttgtgaatg caatttcgag cctaacctat ccaggtgctc tcgtaaaagc gaattcggaa    2040
ttagtagaaa atcaaccaga tgttctccct gtaaaacgtg attcattaac actcagcatt    2100
gatttgccag gtatgactaa tcaagacaat aaaatagttg taaaaaatgc cactaaatca    2160
aacgttaaca acgcagtaaa tacattagtg gaaagatgga atgaaaaata tgctcaagct    2220
tatccaaatg taagtgcaaa aattgattat gatgacgaaa tggcttacag tgaatcacaa    2280

ttaattgcga aatttggtac agcatttaaa gctgtaaata atagcttgaa tgtaaacttc 2340
ggcgcaatca gtgaagggaa aatgcaagaa gaagtcatta gttttaaaca aatttactat 2400
aacgtgaatg ttaatgaacc tacaagacct tccagatttt tcggcaaagc tgttactaaa 2460
gagcagttgc aagcgcttgg agtgaatgca gaaaatcctc ctgcatatat ctcaagtgtg 2520
gcgtatggcc gtcaagttta tttgaaatta tcaactaatt cccatagtac taaagtaaaa 2580
gctgcttttg atgctgccgt aagcggaaaa tctgtctcag gtgatgtaga actaacaaat 2640
atcatcaaaa attcttcctt caaagccgta atttacggag gttccgcaaa agatgaagtt 2700
caaatcatcg acggcaacct cggagactta cgcgatattt tgaaaaaagg cgctactttt 2760
aatcgagaaa caccaggagt tcccattgct tatacaacaa acttcctaaa agacaatgaa 2820
ttagctgtta ttaaaaacaa ctcagaatat attgaaacaa cttcaaaagc ttatacagat 2880
ggaaaaatta acatcgatca ctctggagga tacgttgctc aattcaacat ttcttgggat 2940
gaagtaaatt atgatcctga aggtaacgaa attgttcaac ataaaaactg gagcgaaaac 3000
aataaaagca agctagctca tttcacatcg tccatctatt tgcctggtaa cgcgagaaat 3060
attaatgttt acgctaaaga atgcactggt ttagcttggg aatggtggag aacggtaatt 3120
gatgaccgga acttaccact tgtgaaaaat agaaatatct ccatctgggg caccacgctt 3180
tatccgaaat atagtaataa agtagataat ccaatcgaat aataactgca gctgaaagat 3240
tgcgcggtct acagtcgtga aggccacacc ggtgaacgtg tgcctggcac cattggtttt 3300
gccaccgtgc gtgcaggtga catcgttggt gaacataccg cgatgtttgc cgatattggc 3360
gagcgtctgg agatcaccca taaggcgtcc agccgtatga catttgctaa cggcgcggta 3420
agatcggctt tgtggttgag tggtaaggaa agcggtcttt ttgatatgcg agatgtactt 3480
gatctcaata atttgtaacc acaaaatatt tgttatggtg caaaaataac acatttaatt 3540
tattgattat aaagggcttt aatttttggc ccttttattt ttggtgttat gtttttaaat 3600
tgtctataag tgccaaaaat tacatgtttt gtcttctgtt tttgttgttt taatgtaaat 3660
tttgaccatt tggtccactt ttttctgctc gtttttattt catgcaatct tcttgctgcg 3720
caagcgtttt ccagaacagg ttagatgatc tttttgtcgc ttaatgcctg taaaacatgc 3780
atgagccaca aaataatata aaaaatcccg ccattaagtt gacttttagc gcccatatct 3840
ccagaatgcc gccgtttgcc agaaattcgt cggtaagcag atttgcattg atttacgtca 3900
tcattgtgaa ttaatatgca aataaagtga gtgaatattc tctggagggt gttttgatta 3960
agtcagcgct attggttctg gaagacggaa cccagtttca cggtcgggcc ataggggcaa 4020
caggttcggc ggttggggaa gtcgttttca atacttcaat gaccggttat caagaaatcc 4080
tcactgatcc ttcctattct cgtcaaatcg ttactcttac ttatccccat attggcaatg 4140
tcggcaccaa tgacgccgat gaagaatctt ctcaggtaca tgcacaaggt ctggtgattc 4200
gcgacctgcc gctgattgcc agcaacttcc gtaataccga agacctctct tcttacctga 4260
aacgccataa catcgtggcg attgccgata tcgatacccg taagctgacg cgtttactgc 4320
gcgagaaagg cgcacagaat ggctgcatta tcgcgggcga taacccggat gcggcgctgg 4380
cgttagaaaa agcccgcg 4398

<210> 2
<211> 975
<212> DNA
<213> Artificial sequence

<220>
<223> Séquence flanquante 5′ dapB

<400> 2
cgctcgccca aggggccgct gtgccactgg tacgcgcacc gcgtgatgcg gcatctgtgc 60
acggcgaatc gggaatggct ggctacgact ttgttgagca caaccgaaag ccgctcggga 120
taccggcgtt tctggcgatt cgggatgccc tgatgcgtgc accagagcct gttaccctgg 180
tggccatcgg cccgttaacc aatattgcgc tgttactttc acaatgcccg gaatgcaagc 240
cgtatattcg ccgtctggtg atcatgggtg gttctgccgg acgcggcaac tgtacgccaa 300
acgccgagtt taatattgct gccgatccag aagctgctgc ctgtgtcttc cgcagtggta 360
ttgaaatcgt catgtgcggt ttggatgtca ccaatcaggc aatattaact cctgactatc 420
tctctacact gccgcagtta aaccgtaccg ggaaaatgct tcacgccctg tttagccact 480
accgtagcgg cagtatgcaa agcggcttgc gaatgcacga tctctgcgcc atcgcctggc 540
tggtgcgccc ggacctgttc actctcaaac cctgttttgt ggcagtggaa actcagggcg 600
aatttacctc aggcacgacg gtggttgata tcgacggttg cctgggcaag ccagccaatg 660
tacaggtggc attggatctg gatgtgaaag gcttccagca gtgggtggct gaggtgctgg 720
ctctggcgtc gtaacctgtc acatgttatt ggcatgcagt cattcatcga ctcatgcctt 780
tcactgatat ccctccctgt ttatcattaa tttctaatta tcagcgtttt tggctggcgg 840

cgtagcgatg cgctggttac tctgaaaacg gtctatgcaa attaacaaaa gagaatagct 900
atgcatgatg caaacatccg cgttgccatc gcgggagccg gggggcgtat gggccgccag 960
ttgattcagg cggcg 975

<210> 3
<211> 377
<212> DNA
<213> Artificial sequence

<220>
<223>Séquence 5' dapB

<400> 3
ctggcattag agggcgtgca gttgggcgct gcgctggagc gtgaaggatc ttctttactg 60
ggcagcgacg ccggtgagct ggccggagcc gggaaaacag gcgttaccgt gcaaagcagc 120
ctcgatgcgg taaaagatga ttttgatgtg tttatcgatt ttacccgtcc ggaaggtacg 180
ctgaaccatc tcgctttttg tcgccagcat ggcaaaggga tggtgatcgg cactacgggg 240
tttgacgaag ccggtaaaca agcaattcgt gacgccgctg ccgatattgc gattgtcttt 300
gctgccaatt ttagcgttgg cgttaacgtc atgcttaagc tgctggagaa agcagccaaa 360
gtgatgggtg actacac 377

<210> 4
<211> 1808
<212> DNA
<213> Artificial sequence

<220>
<223> Séquence Ptet hly

<400> 4
tcaaatgtag cacctgaagt cagccccata cgatataagt tgtaattctc atgtttgaca 60
gcttatcatc gataagcttt aatgcggtag tttatcacag ttaaattgct aacgcagtca 120
ggcaccgtgt atgaaatcta acaatgcgct catcgtcatc ctcggcaccg tcaccctgga 180
tgctgtaggc ataggcttgg ttatgccggt actgccgggc ctcttgcggg atatcctcta 240
gaaataattt tgtttaactt taagaaggag atatacatat gaaggatgca tctgcattca 300
ataaagaaaa ttcaatttca tccatggcac caccagcatc tccgcctgca agtcctaaga 360
cgccaatcga aaagaaacac gcggatgaaa tcgataagta tatacaagga ttggattaca 420
ataaaaacaa tgtattagta taccacggag atgcagtgac aaatgtgccg ccaagaaaag 480
gttacaaaga tggaaatgaa tatattgttg tggagaaaaa gaagaaatcc atcaatcaaa 540
ataatgcaga cattcaagtt gtgaatgcaa tttcgagcct aacctatcca ggtgctctcg 600
taaaagcgaa ttcggaatta gtagaaaatc aaccagatgt tctccctgta aaacgtgatt 660
cattaacact cagcattgat ttgccaggta tgactaatca agacaataaa atagttgtaa 720
aaaatgccac taaatcaaac gttaacaacg cagtaaatac attagtggaa agatggaatg 780
aaaaatatgc tcaagcttat ccaaatgtaa gtgcaaaaat tgattatgat gacgaaatgg 840
cttacagtga atcacaatta attgcgaaat ttggtacagc atttaaagct gtaaataata 900
gcttgaatgt aaacttcggc gcaatcagtg aagggaaaat gcaagaagaa gtcattagtt 960
ttaaacaaat ttactataac gtgaatgtta atgaacctac aagaccttcc agatttttcg 1020
gcaaagctgt tactaaagag cagttgcaag cgcttggagt gaatgcagaa aatcctcctg 1080
catatatctc aagtgtggcg tatggccgtc aagtttattt gaaattatca actaattccc 1140
atagtactaa agtaaaagct gcttttgatg ctgccgtaag cggaaaatct gtctcaggtg 1200
atgtagaact aacaaatatc atcaaaaatt cttccttcaa agccgtaatt tacggaggtt 1260
ccgcaaaaga tgaagttcaa atcatcgacg gcaacctcgg agacttacgc gatattttga 1320
aaaaaggcgc tacttttaat cgagaaacac caggagttcc cattgcttat acaacaaact 1380
tcctaaaaga caatgaatta gctgttatta aaaacaactc agaatatatt gaaacaactt 1440
caaaagctta tacagatgga aaaattaaca tcgatcactc tggaggatac gttgctcaat 1500
tcaacatttc ttgggatgaa gtaaattatg atcctgaagg taacgaaatt gttcaacata 1560
aaaactggag cgaaaacaat aaaagcaagc tagctcattt cacatcgtcc atctatttgc 1620
ctggtaacgc gagaaatatt aatgtttacg ctaaagaatg cactggttta gcttgggaat 1680
ggtggagaac ggtaattgat gaccggaact taccacttgt gaaaaataga aatatctcca 1740

tctggggcac cacgctttat ccgaaatata gtaataaagt agataatcca atcgaataat 1800
aactgcag 1808

<210> 5
<211> 490
<212> DNA
<213> Artificial sequence

<220>
<223> Séquence 3'dapB

<400> 5
ctgaaagatt gcgcggtcta cagtcgtgaa ggccacaccg gtgaacgtgt gcctggcacc 60
attggttttg ccaccgtgcg tgcaggtgac atcgttggtg aacataccgc gatgtttgcc 120
gatattggcg agcgtctgga gatcacccat aaggcgtcca gccgtatgac atttgctaac 180
ggcgcggtaa gatcggcttt gtggttgagt ggtaaggaaa gcggtctttt tgatatgcga 240
gatgtacttg atctcaataa tttgtaacca caaaatattt gttatggtgc aaaaataaca 300
catttaattt attgattata aagggcttta atttttggcc cttttatttt tggtgttatg 360
tttttaaatt gtctataagt gccaaaaatt acatgttttg tcttctgttt ttgttgtttt 420
aatgtaaatt ttgaccattt ggtccacttt tttctgctcg tttttatttc atgcaatctt 480
cttgctgcgc 490

<210> 6
<211> 677
<212> DNA
<213> Artificial sequence

<220>
<223> Séquence flanquante 3' dapB
<400> 6
aagcgttttc cagaacaggt tagatgatct ttttgtcgct taatgcctgt aaaacatgca 60
tgagccacaa aataatataa aaaatcccgc cattaagttg acttttagcg cccatatctc 120
cagaatgccg ccgtttgcca gaaattcgtc ggtaagcaga tttgcattga tttacgtcat 180
cattgtgaat taatatgcaa ataaagtgag tgaatattct ctggagggtg ttttgattaa 240
gtcagcgcta ttggttctgg aagacggaac ccagtttcac ggtcgggcca taggggcaac 300
aggttcggcg gttggggaag tcgttttcaa tacttcaatg accggttatc aagaaatcct 360
cactgatcct tcctattctc gtcaaatcgt tactcttact tatccccata ttggcaatgt 420
cggcaccaat gacgccgatg aagaatcttc tcaggtacat gcacaaggtc tggtgattcg 480
cgacctgccg ctgattgcca gcaacttccg taataccgaa gacctctctt cttacctgaa 540
acgccataac atcgtggcga ttgccgatat cgatacccgt aagctgacgc gtttactgcg 600
cgagaaaggc gcacagaatg gctgcattat cgcgggcgat aacccggatg cggcgctggc 660
gttagaaaaa gcccgcg 677

<210> 7
<211> 5863
<212> DNA
<213> Artificial sequence

<220>
<223> Séquence « Delta dapA oméga inv » de BM4570

<400> 7
   tttcaactca tccatgttat cgcgtaagcc gcaggcctgt acggtacagc cgggggtcat 60
   ggctttcggg tagaaataaa ccagaacacg ctgtccctgg aagtcggtca aattaacttg 120
   ttctccgtct tgatccggca agctaaattt cggtgcgata tcaccggctt tcagtggatt 180
   cattacttaa ctccatcctg ttcatcatgt tgggaataat tgacgacgtt aatactgcct 240
   tgtgcattga gttctgtaca tagggctttg aacgcttgct caatatttgc tgcgtccgca 300
   gatgcggggc tgtgggcggt tatctgaata tgcaactgcg cagcccgttc attttcagca 360
   ggttgcgtgc gcgacaccag ctccgcaatg ttcatatgat gcgcgtcgaa aagtgctgtg 420
   aagcgttcaa ttaaatgcgg ggagtctgcc acatcgacct gaacccagac agatgctggc 480
   attggcggac gcggacgcgc cgtcgtgcgc ttcatcacga ttaaaagatc cagttcggca 540
   cctttcaacg gtaacgttga ttcaatcaga gtaatggcat tccatgaacc ggaaagcagc 600
   ataataaacg tgaactcttc tcccagcatc gccaggcgac tgtcttcaat attacagccg 660
   caactactga catgacgggt gatggtgttc acaattccag ggcgatcggc acccaacgca 720
   gtgatcacca gataatgttg cgatgacagt gtcaaactgg ttattccttt aaggggtgag 780
   ttgttcttaa ggaaagcata aaaaaaacat gcatacaaca atcagaacgg ttctgtctgc 840
   ttgcttttaa tgccatacca aacgtaccat tgagacactt gtttgcacag aggatggccc 900
   atgttcacgg gaagtattgt cgcgattgtt actccgatgg atgaaaaagg taatgtctgt 960
   cgggctagct tgaaaaaact gattgattat catgtcgcca gcggtacttc ggcgatcgtt 1020
   tctgttggca ccactggcga gtccgctacc ttaaatcatg acgaacatgc tgatgtggtg 1080
   atgatgacgc tggatctggc tgatgggcgc attccggtaa ttgccgggac cggcgctaac 1140
   gctactgcgg aagccattag cctgacgcag cgcttcaatg acagtggtat cgtcggctgc 1200
   ctgacggtaa ccccttacta caatcgtccg tcgcaagaag gtttgtatca gcatttcaaa 1260
   gccatcgctg agcatactga cctgccgcaa attctgtata atgtgccgtc ccgtactggc 1320
   tgcgatctgc tcccggaaac ggtgggccgt ctggcgaaag taaaaaatat tatcggaatc 1380
   aaagaggcaa cagggaactt aactagataa ctgaggatct gaagttccta ttccgaagtt 1440
   cctattctct agaaagtata ggaacttcag atccctggca aatattctga aatgagctgt 1500
   tgacaattaa tcatccggct cgtataatgt gtggaattgt gagcggataa caattgcagt 1560
   ttgacgtatg acaggtatgc tttatttcat ttaaattatg atggttttcc agccaatcag 1620
   tgagtttctc ttgataagga atgcgggaat gtctatgtat tttaataaaa taatttcatt 1680
   taatattatt tcacgaatag ttatttgtat ctttttgata tgtggaatgt tcatggctgg 1740
   ggcttcagaa aaatatgatg ctaacgcacc gcaacaggtc cagccttatt ctgtctcttc 1800
   atctgcattt gaaaatctcc atcctaataa tgaaatggag agttcaatca atcccttttc 1860
   cgcatcggat acagaaagaa atgctgcaat aatagatcgc gccaataagg agcaggagac 1920
   tgaagcggtg aataagatga taagcaccgg ggccaggtta gctgcatcag gcagggcatc 1980
   tgatgttgct cactcaatgg tgggcgatgc ggttaatcaa gaaatcaaac agtggttaaa 2040
   tcgattcggt acggctcaag ttaatctgaa ttttgacaaa aatttttcgc taaaagaaag 2100
   ctctcttgat tggctggctc cttggtatga ctctgcttca ttcctctttt ttagtcagtt 2160
   aggtattcgc aataaagaca gccgcaacac acttaacctt ggcgtcggga tacgtacatt 2220
   ggagaacggt tggctgtacg gacttaatac tttttatgat aatgatttga ccggccacaa 2280
   ccaccgtatc ggtcttggtg ccgaggcctg gaccgattat ttacagttgg ctgccaatgg 2340
   gtattttcgc ctcaatggat ggcactcgtc gcgtgatttc tccgactata aagagcgccc 2400
   agccactggg ggggatttgc gcgcgaatgc ttatttacct gcactcccac aactgggggg 2460
   gaagttgatg tatgagcaat acaccggtga gcgtgttgct ttatttggta aagataatct 2520
   gcaacgcaac ccttatgccg tgactgccgg gatcaattac acccccgtgc ctctactcac 2580
   tgtcggggta gatcagcgta tggggaaaag cagtaagcat gaaacacagt ggaacctcca 2640
   aatgaactat cgcctgggcg agagttttca gtcgcaactt agcccttcag cggtggcagg 2700
   aacacgtcta ctggcggaga gccgctataa ccttgtcgat cgtaacaata atatcgtgtt 2760
   ggagtatcag aaacagcagg tggttaaact gacattatcg ccagcaacta tctccggcct 2820
   gccgggtcag gtttatcagg tgaacgcaca agtacaaggg gcatctgctg taagggaaat 2880
   tgtctggagt gatgccgaac tgattgccgc tggcggcaca ttaacaccac tgagtaccac 2940
   acaattcaac ttggttttac cgccttataa acgcacagca caagtgagtc gggtaacgga 3000
   cgacctgaca gccaactttt attcgcttag tgcgctcgcg gttgatcacc aaggaaaccg 3060
   atctaactca ttcacattga gcgtcaccgt tcagcagcct cagttgacat taacggcggc 3120
   cgtcattggt gatggcgcac cggctaatgg gaaaactgca atcaccgttg agttcaccgt 3180
   tgctgatttt gaggggaaac ccttagccgg gcaggaggtg gtgataacca ccaataatgg 3240
   tgcgctaccg aataaaatca cggaaaagac agatgcaaat ggcgtcgcgc gcattgcatt 3300
   aaccaatacg acagatggcg tgacggtagt cacagcagaa gtggaggggc aacggcaaag 3360
   tgttgatacc cactttgtta agggtactat cgcggcggat aaatccactc tggctgcggt 3420
   accgacatct atcatcgctg atggtctaat ggcttcaacc atcacgttgg agttgaagga 3480
   tacctatggg gacccgcagg ctggcgcgaa tgtggctttt gacacaacct taggcaatat 3540
   gggcgttatc acggatcaca atgacggcac ttatagcgca ccattgacca gtaccacgtt 3600
   gggggtagca acagtaacgg tgaaagtgga tggggctgcg ttcagtgtgc cgagtgtgac 3660
   ggttaatttc acggcagatc ctattccaga tgctggccgc tccagtttca ccgtctccac 3720
   accggatatc ttggctgatg gcacgatgag ttccacatta tcctttgtcc ctgtcgataa 3780
   gaatggccat tttatcagtg ggatgcaggg cttgagtttt actcaaaacg gtgtgccggt 3840
   gagtattagc cccattaccg agcagccaga tagctatacc gcgacggtgg ttgggaatag 3900
   tgtcggtgat gtcacaatca cgccgcaggt tgataccctg atactgagta cattgcagaa 3960
   aaaaatatcc ctattcccgg tacctacgct gaccggtatt ctggttaacg ggcaaaattt 4020
   cgctacggat aaagggttcc cgaaaacgat ctttaaaaac gccacattcc agttacagat 4080
   ggataacgat gttgctaata atactcagta tgagtggtcg tcgtcattca cacccaatgt 4140
   atcggttaac gatcagggtc aggtgacgat tacctaccaa acctatagcg aagtggctgt 4200
   gacggcgaaa agtaaaaaat tcccaagtta ttcggtgagt tatcggttct acccaaatcg 4260
   gtggatatac gatggcggca gatcgctggt atccagtctc gaggccagca gacaatgcca 4320
   aggttcagat atgtctgcgg ttcttgaatc ctcacgtgca accaacggaa cgcgtgcgcc 4380
   tgacgggaca ttgtggggcg agtgggggag cttgaccgcg tatagttctg attggcaatc 4440
   tggtgaatat tgggtcaaaa agaccagcac ggattttgaa accatgaata tggacacagg 4500
   cgcactgcaa ccagggcctg catacttggc gttcccgctc tgtgcgctgt caatataacc 4560
   agataacaga tagcaataag aacagtttaa tgagctgatt atttggggcg cgaatgggag 4620
   tccggcaatc ctagactcgc cccataagta gcaaacgtcc agagaacaac gccgctcagg 4680
   ttaattgagc ggcgttgttt ttttaaaagg atttgtcgcg ataagcgtga gctggcgtta 4740
   aatgccgatc ttacggccca ggatattggc actgcagatt aatcagcgtc tgatgccatt 4800
   acacaacaaa ctatttgtcg aacccaatcc aatcccggtg aaatgggcat gtaaggaact 4860
   gggtcttgtg gcgaccgata cgctgcgcct gccaatgaca ccaatcaccg acagtggtcg 4920
   tgagacggtc agagcggcgc ttaagcatgc cggtttgctg taaagtttag ggagatttga 4980
   tggcttactc tgttcaaaag tcgcgcctgg caaaggttgc gggtgtttcg cttgttttat 5040
   tactcgctgc ctgtagttct gactcacgct ataagcgtca ggtcagtggt gatgaagcct 5100
   acctggaagc ggcaccgctt gcggagcttc atgccccggc tggaatgatt ttgccggtga 5160
   cctccggtga ttatgcaatc ccggtgacca acggtagtgg tgctgtcggt aaggcgctgg 5220
   acattcgtcc accagcccag ccgctggcac tggtttctgg cgcgcgtacc cagttcacgg 5280
   gcgataccgc ttcattgctg gtggaaaatg gtcgtggcaa tactctgtgg ccgcaggtgg 5340
   ttagcgtgct gcaggcgaaa aactacacca tcacccaacg tgatgatgct ggtcagacac 5400
   tgaccaccga ttgggtacaa tggaaccgtc tggacgaaga cgagcagtat cgtggtcgtt 5460
   atcaaatctc tgttaagccg cagggttatc agcaggcggt tacggttaaa ctgctgaacc 5520
   tggaacaggc gggcaaaccg gttgcagacg cggcttccat gcagcgttac agcacggaga 5580
   tgatgaacgt tatttccgcc ggtctggata aatctgccac tgacgccgcg aacgctgcgc 5640
   aaaatcgtgc ctccaccact atggacgtac aaagtgcagc tgatgacacc ggtttaccaa 5700
   tgctggtcgt acgcgggccg ttcaatgtgg tttggcaacg tctgccagcg gcgctggaaa 5760
   aagtgggcat gaaagtgacc gacagcaccc gttcgcaggg caacatggcc gtaacttata 5820
   agccgctgtc tgacagcgac tggcaggaac tgggcgcgag cga 5863

<210> 8
<211> 993
<212> DNA
<213> Artificial sequence

<220>
<223> Séquence flanquante 5' de dapA

<400> 8
tttcaactca tccatgttat cgcgtaagcc gcaggcctgt acggtacagc cgggggtcat 60
ggctttcggg tagaaataaa ccagaacacg ctgtccctgg aagtcggtca aattaacttg 120
ttctccgtct tgatccggca agctaaattt cggtgcgata tcaccggctt tcagtggatt 180
cattacttaa ctccatcctg ttcatcatgt tgggaataat tgacgacgtt aatactgcct 240
tgtgcattga gttctgtaca tagggctttg aacgcttgct caatatttgc tgcgtccgca 300
gatgcggggc tgtgggcggt tatctgaata tgcaactgcg cagcccgttc attttcagca 360
ggttgcgtgc gcgacaccag ctccgcaatg ttcatatgat gcgcgtcgaa aagtgctgtg 420
aagcgttcaa ttaaatgcgg ggagtctgcc acatcgacct gaacccagac agatgctggc 480
attggcggac gcggacgcgc cgtcgtgcgc ttcatcacga ttaaaagatc cagttcggca 540
cctttcaacg gtaacgttga ttcaatcaga gtaatggcat tccatgaacc ggaaagcagc 600
ataataaacg tgaactcttc tcccagcatc gccaggcgac tgtcttcaat attacagccg 660
caactactga catgacgggt gatggtgttc acaattccag ggcgatcggc acccaacgca 720
gtgatcacca gataatgttg cgatgacagt gtcaaactgg ttattccttt aaggggtgag 780
ttgttcttaa ggaaagcata aaaaaaacat gcatacaaca atcagaacgg ttctgtctgc 840
ttgcttttaa tgccatacca aacgtaccat tgagacactt gtttgcacag aggatggccc 900
atgttcacgg gaagtattgt cgcgattgtt actccgatgg atgaaaaagg taatgtctgt 960
cgggctagct tgaaaaaact gattgattat cat 993

<210> 9
<211> 410
<212> DNA
<213> Artificial sequence

<220>
<223> Séquence 5' de dapA

<400> 9
gtcgccagcg gtacttcggc gatcgtttct gttggcacca ctggcgagtc cgctacctta 60
aatcatgacg aacatgctga tgtggtgatg atgacgctgg atctggctga tgggcgcatt 120
ccggtaattg ccgggaccgg cgctaacgct actgcggaag ccattagcct gacgcagcgc 180
ttcaatgaca gtggtatcgt cggctgcctg acggtaaccc cttactacaa tcgtccgtcg 240
caagaaggtt tgtatcagca tttcaaagcc atcgctgagc atactgacct gccgcaaatt 300
ctgtataatg tgccgtcccg tactggctgc gatctgctcc cggaaacggt gggccgtctg 360
gcgaaagtaa aaaatattat cggaatcaaa gaggcaacag ggaacttaac 410

<210> 10
<211> 3303
<212> DNA
<213> Artificial sequence

<220>
<223> Séquence du Ptrc inv

<400> 10
ctggcaaata ttctgaaatg agctgttgac aattaatcat ccggctcgta taatgtgtgg 60
aattgtgagc ggataacaat tgcagtttga cgtatgacag gtatgcttta tttcatttaa 120
attatgatgg ttttccagcc aatcagtgag tttctcttga taaggaatgc gggaatgtct 180
atgtatttta ataaaataat ttcatttaat attatttcac gaatagttat ttgtatcttt 240
ttgatatgtg gaatgttcat ggctggggct tcagaaaaat atgatgctaa cgcaccgcaa 300
caggtccagc cttattctgt ctcttcatct gcatttgaaa atctccatcc taataatgaa 360
atggagagtt caatcaatcc cttttccgca tcggatacag aaagaaatgc tgcaataata 420
gatcgcgcca ataaggagca ggagactgaa gcggtgaata agatgataag caccggggcc 480
aggttagctg catcaggcag ggcatctgat gttgctcact caatggtggg cgatgcggtt 540
aatcaagaaa tcaaacagtg gttaaatcga ttcggtacgg ctcaagttaa tctgaatttt 600
gacaaaaatt tttcgctaaa agaaagctct cttgattggc tggctccttg gtatgactct 660
gcttcattcc tcttttttag tcagttaggt attcgcaata aagacagccg caacacactt 720
aaccttggcg tcgggatacg tacattggag aacggttggc tgtacggact taatactttt 780
tatgataatg atttgaccgg ccacaaccac cgtatcggtc ttggtgccga ggcctggacc 840
gattatttac agttggctgc caatgggtat tttcgcctca atggatggca ctcgtcgcgt 900
gatttctccg actataaaga gcgcccagcc actggggggg atttgcgcgc gaatgcttat 960
ttacctgcac tcccacaact gggggggaag ttgatgtatg agcaatacac cggtgagcgt 1020
gttgctttat ttggtaaaga taatctgcaa cgcaaccctt atgccgtgac tgccgggatc 1080
aattacaccc ccgtgcctct actcactgtc ggggtagatc agcgtatggg gaaaagcagt 1140
aagcatgaaa cacagtggaa cctccaaatg aactatcgcc tgggcgagag ttttcagtcg 1200
caacttagcc cttcagcggt ggcaggaaca cgtctactgg cggagagccg ctataacctt 1260
gtcgatcgta acaataatat cgtgttggag tatcagaaac agcaggtggt taaactgaca 1320
ttatcgccag caactatctc cggcctgccg ggtcaggttt atcaggtgaa cgcacaagta 1380
caaggggcat ctgctgtaag ggaaattgtc tggagtgatg ccgaactgat tgccgctggc 1440
ggcacattaa caccactgag taccacacaa ttcaacttgg ttttaccgcc ttataaacgc 1500
acagcacaag tgagtcgggt aacggacgac ctgacagcca acttttattc gcttagtgcg 1560
ctcgcggttg atcaccaagg aaaccgatct aactcattca cattgagcgt caccgttcag 1620
cagcctcagt tgacattaac ggcggccgtc attggtgatg gcgcaccggc taatgggaaa 1680
actgcaatca ccgttgagtt caccgttgct gattttgagg ggaaaccctt agccgggcag 1740
gaggtggtga taaccaccaa taatggtgcg ctaccgaata aaatcacgga aaagacagat 1800
gcaaatggcg tcgcgcgcat tgcattaacc aatacgacag atggcgtgac ggtagtcaca 1860
gcagaagtgg aggggcaacg gcaaagtgtt gatacccact ttgttaaggg tactatcgcg 1920
gcggataaat ccactctggc tgcggtaccg acatctatca tcgctgatgg tctaatggct 1980

tcaaccatca cgttggagtt gaaggatacc tatggggacc cgcaggctgg cgcgaatgtg 2040
gcttttgaca caaccttagg caatatgggc gttatcacgg atcacaatga cggcacttat 2100
agcgcaccat tgaccagtac cacgttgggg gtagcaacag taacggtgaa agtggatggg 2160
gctgcgttca gtgtgccgag tgtgacggtt aatttcacgg cagatcctat tccagatgct 2220
ggccgctcca gtttcaccgt ctccacaccg gatatcttgg ctgatggcac gatgagttcc 2280
acattatcct ttgtccctgt cgataagaat ggccatttta tcagtgggat gcagggcttg 2340
agttttactc aaaacggtgt gccggtgagt attagcccca ttaccgagca gccagatagc 2400
tataccgcga cggtggttgg gaatagtgtc ggtgatgtca caatcacgcc gcaggttgat 2460
accctgatac tgagtacatt gcagaaaaaa atatccctat tcccggtacc tacgctgacc 2520
ggtattctgg ttaacgggca aaatttcgct acggataaag ggttcccgaa aacgatcttt 2580
aaaaacgcca cattccagtt acagatggat aacgatgttg ctaataatac tcagtatgag 2640
tggtcgtcgt cattcacacc caatgtatcg gttaacgatc agggtcaggt gacgattacc 2700
taccaaacct atagcgaagt ggctgtgacg gcgaaaagta aaaaattccc aagttattcg 2760
gtgagttatc ggttctaccc aaatcggtgg atatacgatg gcggcagatc gctggtatcc 2820
agtctcgagg ccagcagaca atgccaaggt tcagatatgt ctgcggttct tgaatcctca 2880
cgtgcaacca acggaacgcg tgcgcctgac gggacattgt ggggcgagtg ggggagcttg 2940
accgcgtata gttctgattg gcaatctggt gaatattggg tcaaaaagac cagcacggat 3000
tttgaaacca tgaatatgga cacaggcgca ctgcaaccag ggcctgcata cttggcgttc 3060
ccgctctgtg cgctgtcaat ataaccagat aacagatagc aataagaaca gtttaatgag 3120
ctgattattt ggggcgcgaa tgggagtccg gcaatcctag actcgcccca taagtagcaa 3180
acgtccagag aacaacgccg ctcaggttaa ttgagcggcg ttgttttttt aaaaggattt 3240
gtcgcgataa gcgtgagctg gcgttaaatg ccgatcttac ggcccaggat attggcactg 3300
cag 3303

<210> 11
<211> 403
<212> DNA
<213> Artificial sequence

<220>
<223> Séquence 3' de dapA

<400> 11
attaatcagc gtctgatgcc attacacaac aaactatttg tcgaacccaa tccaatcccg 60
gtgaaatggg catgtaagga actgggtctt gtggcgaccg atacgctgcg cctgccaatg 120
acaccaatca ccgacagtgg tcgtgagacg gtcagagcgg cgcttaagca tgccggtttg 180
ctgtaaagtt tagggagatt tgatggctta ctctgttcaa aagtcgcgcc tggcaaaggt 240
tgcgggtgtt tcgcttgttt tattactcgc tgcctgtagt tctgactcac gctataagcg 300
tcaggtcagt ggtgatgaag cctacctgga agcggcaccg cttgcggagc ttcatgcccc 360
ggctggaatg attttgccgg tgacctccgg tgattatgca atc 403

<210> 12
<211> 683
<212> DNA
<213> Artificial sequence

<220>
<223> Séquence flanquante 3' de dapA

<400> 12
ccggtgacca acggtagtgg tgctgtcggt aaggcgctgg acattcgtcc accagcccag 60
ccgctggcac tggtttctgg cgcgcgtacc cagttcacgg gcgataccgc ttcattgctg 120
gtggaaaatg gtcgtggcaa tactctgtgg ccgcaggtgg ttagcgtgct gcaggcgaaa 180
aactacacca tcacccaacg tgatgatgct ggtcagacac tgaccaccga ttgggtacaa 240
tggaaccgtc tggacgaaga cgagcagtat cgtggtcgtt atcaaatctc tgttaagccg 300
cagggttatc agcaggcggt tacggttaaa ctgctgaacc tggaacaggc gggcaaaccg 360
gttgcagacg cggcttccat gcagcgttac agcacggaga tgatgaacgt tatttccgcc 420
ggtctggata aatctgccac tgacgccgcg aacgctgcgc aaaatcgtgc ctccaccact 480
atggacgtac aaagtgcagc tgatgacacc ggtttaccaa tgctggtcgt acgcgggccg 540

ttcaatgtgg tttggcaacg tctgccagcg gcgctggaaa aagtgggcat gaaagtgacc 600
gacagcaccc gttcgcaggg caacatggcc gtaacttata agccgctgtc tgacagcgac 660
tggcaggaac tgggcgcgag cga 683

## Description

La présente invention concerne un système vectoriel capable de délivrer dans des cellules cibles eucaryotes un acide nucléique d'intérêt, ou codant pour une protéine ce système vectoriel comprenant une bactérie *Escherichia coli* (*E. coli*) recombinante non pathogène ayant intégré de façon ciblée et sans marqueur antibiotique dans son chromosome un ou plusieurs gènes conférant cette bactérie *E. coli* la capacité de pénétrer dans le cytoplasme de ces cellules cibles eucaryotes. La présente invention comprend également l'utilisation de telles bactéries *E. coli* pour délivrer des compositions thérapeutiques destinées à la prévention ou au traitement de maladie par vaccination ou par thérapie génique.

Le transfert de matériel génétique dans des cellules de mammifères au moyen de bactérie *E. coli* invasive non pathogène a déjà fait l'objet de publications (demande de brevet français FR 95 15556 publiée sous le numéro 2 743 086 ; Grillot-Courvalin C., Goussard S., Huetz F., Ojcius D.M., and Courvalin P. (1998), Nature Biotechnol., 16:862-866).

Les techniques décrites dans ces documents concernent une bactérie *E. coli* modifiée génétiquement dans le but de pénétrer et d'atteindre le cytoplasme de cellules eucaryotes cibles déterminées, d'empêcher sa multiplication et sa survie dans les cellules cibles, dès son entrée dans le cytoplasme de ces cellules eucaryotes cibles. Cette bactérie est en outre transformée par un plasmide codant pour un gène, que l'on souhaite transférer dans la cellule cible, qui est un vecteur navette procaryote-eucaryote qui peut être réplicatif dans ladite bactérie *E. coli,* ou encore intégratif dans lesdites cellules eucaryotes hôtes cibles.

Plus précisément, ces documents décrivent de telles bactéries *E. coli* dans lesquelles les gènes conférant à la bactérie *E. coli* sa capacité à pénétrer et à atteindre le cytoplasme des cellules eucaryotes cibles, tels que le gène d'invasion *inv* de *Yersinia pseudotuberculosis* (*Y. pseudotuberculosis*), et le gène *hly* codant pour l'hémolysine de *Listeria monocytogenes* (*L. monocytogenes*) sont apportés par des plasmides (type pGB2Ω*inv-h*/y). Parmi les bactéries *E. coli* recombinantes, non pathogènes et invasives ainsi transformées, la souche *E. coli* BM2710/pGB2Ω*inv-hly* est particulièrement décrite (voir également Courvalin et al., CR Acad. Sci., 1995, 318, pages 1207-1212). Cette souche BM2710 a été déposée à la CNCM (Collection Nationale de Cultures de Microorganismes, Institut Pasteur, 25 Rue du Docteur Roux, 75724 Paris Cédex 15, France), le 27 octobre 1995 sous le numéro I-1635.

Ces travaux initiaux ont permis de montrer que la souche de *E. coli* BM2710 auxotrophe pour l'acide diaminopimélique (qui se lyse sans se diviser par défaut de synthèse de sa paroi dans un environnement déficient en dap comme c'est le cas dans les cellules eucaryotes) et rendue invasive était capable de délivrer de l'ADN plasmidique à différents types cellulaires chez lesquels l'expression du transgène a pu être détectée (Courvalin et al., 1995). Pour un transfert efficace, deux étapes sont apparues nécessaires : l'entrée de la bactérie dans la cellule et sa sortie (ou celle de l'ADN plasmidique) de la vacuole de phagocytose induite. L'internalisation du vecteur bactérien dans de nombreux types cellulaires a été rendue possible par l'introduction dans *E. coli* BM2710 *dap* du gène *inv* de *Y. pseudotuberculosis.* Ce gène dirige la synthèse de l'invasine qui se lie avec une haute affinité à des récepteurs de la membrane cellulaire, les β1-intégrines. La sortie de la vacuole de phagocytose est contrôlée par l'acquisition par *E. coli* BM2710 du gène *hly* de *L. monocytogenes* qui dirige la synthèse de la listériolysine O. La listériolysine O permet à *E. coli,* ou à son contenu plasmidique en cas de lyse de la bactérie, de sortir de la vacuole de phagocytose grâce à la formation de pores membranaires. Dans cette première génération du vecteur bactérien, les gènes *inv* et *hly* ont été clonés dans le plasmide à bas nombre de copies pGB2 qui confère la résistance à la spectinomycine et à la streptomycine. Il a été montré que ce vecteur bactérien permet, par invasion abortive, de transférer et de faire exprimer par des cellules de mammifères des vecteurs intégratifs ou réplicatifs avec une bonne efficacité (5 à 20 % des cellules en fonction du type cellulaire transfecté *in vitro* : cellules HeLa, CHO ou COS-1). L'efficacité de transfert du plasmide d'intérêt est évaluée à l'aide d'un gène rapporteur codant pour la « green fluorescent protein » (GFP) placé sous le contrôle d'un promoteur eucaryote. Le transfert s'observe également, mais avec une efficacité plus faible, en l'absence de division cellulaire ou lorsque l'invasion est réalisée à confluence cellulaire (Grillot-Courvalin et al., 1998).

Les avantages du transfert de gène par invasion bactérienne abortive résident dans la faible toxicité pour les cellules réceptrices, la rareté des remaniements dans l'ADN délivré, la bonne efficacité de transfection et également dans le fait que la bactérie donatrice, non pathogène et bien définie génétiquement, est incapable de se multiplier et même de survivre dans la cellule réceptrice et dans l'environnement du fait de l'auxotrophie pour l'acide diaminopimélique.

Parmi les améliorations à apporter, on peut citer notamment la possibilité d'introduire n'importe quel vecteur plasmidique d'expression sans avoir à respecter les règles de compatibilité des origines de réplication et également la possibilité de diminuer voire de supprimer les gènes de résistance aux antibiotiques présents dans la bactérie vectrice.

Les inventeurs ont mis en évidence qu'il était possible d'obtenir de telles améliorations en intégrant dans le chromosome bactérien les gènes conférant à la bactérie *E. coli* sa capacité à pénétrer et à atteindre le cytoplasme des cellules eucaryotes cibles, tels que les gènes *inv* et *hly*.

Les inventeurs ont ainsi généré une souche *E. coli* qui héberge ces deux gènes dans le chromosome.

En dehors des avantages précités, cette intégration chromosomique permet de stabiliser les gènes *inv* et *hly* dans la progénie.

La présente invention a donc pour objet un système vectoriel capable de délivrer dans des cellules cibles eucaryotes un acide nucléique d'intérêt ou codant pour une protéine d'intérêt, système vectoriel comprenant une bactérie *E. coli* recombinante non pathogène, ladite bactérie *E. coli* non pathogène étant en outre :
- modifiée par introduction (par échange allélique) d'un ou plusieurs gènes conférant à cette bactérie *E. coli* recombinante non pathogène la capacité de pénétrer dans le cytoplasme desdites cellules cibles eucaryotes ;
- incapable de survivre dans le cytoplasme de ladite cellule cible ; et
- modifiée par les fragments d'ADN étrangers,
**caractérisé en ce que** le ou les gènes conférant à ladite bactérie *E. coli* recombinante non pathogène la capacité de pénétrer dans le cytoplasme desdites cellules cibles eucaryotes sont intégrés dans le chromosome de ladite *E. coli* non pathogène.

Les expressions « système vectoriel » et « cellule cibles » reflètent que la souche *E. coli* est utilisée ici comme moyen de transfert de l'acide nucléique d'intérêt dans lesdites cellules eucaryotes et non comme bactérie hôte pour l'expression dudit fragment d'ADN, celle-ci pouvant avoir lieu le cas échéant dans lesdites cellules cibles après pénétration de la bactérie dans le cytoplasme. Ce système vectoriel couvre également les souches recombinantes de *E. coli* telles que définies en tant que système vectoriel et défini dans la présente invention.

Par « acide nucléique d'intérêt » on entend désigner ici toute séquence nucléotidique, ADN ou ARN, comportant ou non des sites de restriction enzymatique. La plupart des systèmes de transfert de matériel génétique ne permettant pas de transférer de grands fragments d'ADN, les vecteurs viraux ne peuvent s'accommoder de fragments de plus de 150 kb ; par lipofection il faut d'abord produire de grandes quantités de ces grands fragments à partir de cultures des bactéries qui répliquent ces chromosomes artificiels bactériens, puis purifier l'ADN et l'introduire dans les cellules par lipofection, toutes ces étapes entraînent souvent une dénaturation du matériel génétique et résultent en une faible efficacité de transfection.

De manière préférentielle, le vecteur bactérien permet de propager et de transférer directement dans le cytoplasme des cellules ces chromosomes artificiels bactériens de toutes tailles sans purification préalable et donc en conservant leur intégrité physique.

Ainsi, dans un mode de réalisation également préféré, l'acide nucléique d'intérêt peut-être de toute taille jusqu'à 100 kb, ou de 100 à 150kb mais aussi plus grand que 150kb la limite supérieure n'étant pas définie.

Dans un mode de réalisation particulièrement préféré, l'acide nucléique comprend au moins 150 kb.

Par « acide nucléique d'intérêt » on entend préférer ici toute séquence nucléotidique hétérologue à la cellule cible ou toute séquence nucléotidique présente dans la cellule cible mais dont on souhaite modifier ou réguler l'expression. De préférence ainsi, il peut s'agir d'un acide nucléique exogène.

Dans un mode de réalisation préféré, le système vectoriel selon l'invention est **caractérisé en ce que** ladite bactérie *E. coli* non pathogène est transformée par intégration chromosomique de deux gènes, provenant d'une ou de deux autres bactéries, lui conférant la capacité de pénétrer dans le cytoplasme desdites cellules cibles.

Dans un mode de réalisation préféré, le système vectoriel selon l'invention est **caractérisé en ce que** le ou les gènes intégrés dans le chromosome de la bactérie *E. coli* non pathogène et lui conférant sa capacité de pénétrer dans le cytoplasme desdites cellules cibles, lui permettent de lyser la membrane des vacuoles desdites cellules cibles.

Lorsque la spécificité de pilotage vers lesdites cellules cibles particulières est conférée par un gène provenant d'une autre bactérie codant pour la faculté de pénétrer spécifiquement lesdites cellules cibles, c'est le choix du gène responsable de la reconnaissance et pénétration de la membrane cellulaire qui confère la spécificité du pilotage. Lesdites cellules eucaryotes cibles peuvent être des cellules animales, notamment des cellules de mammifères, en particulier des cellules humaines, ou des cellules de levures ou encore des cellules de plantes.

La nature de la bactérie d'origine du gène responsable de la reconnaissance et pénétration spécifique dans les cellules cibles déterminées, utilisé selon la présente invention, confère au système vectoriel de l'invention, une spécificité de pilotage vers un type de cellules eucaryotes cibles, en particulier des cellules de mammifères notamment humaines, correspondant au spectre d'hôte cellulaire de ladite bactérie.

Parmi les gènes conférant aux bactéries capacité de pénétrer dans certaines cellules cibles, on peut citer :
- un gène ou un ensemble de gènes de la bactérie *Salmonella typhimurium* pour transférer de l'ADN dans des cellules de tissus lymphoïdes et des cellules hépatiques ;
- un gène ou un ensemble de gènes de la bactérie *Shigella flexneri* pour transférer de l'ADN dans des cellules épithéliales de mammifères ;
- un gène de la bactérie du genre Légionella, notamment *Legionella pneumophila*, notamment pour transférer de l'ADN plus spécifiquement dans des cellules épithéliales du poumon ;
- un gène de la bactérie *L. monocytogenes* pour transférer de l'ADN dans des cellules épithéliales du système nerveux central ;
- un gène de la bactérie du genre Yersinia, notamment *Y. pseudotuberculosis* ou *Y. enterocolitica* pour transférer de l'ADN dans des cellules épithéliales ; et
- un gène de bactérie du genre Mycobactérium, notamment *Mycobactérium tuberculosis, Avilum complex*, scrofulaceum pour transférer de l'ADN dans des macrophages.

Dans un mode de réalisation illustrant cet aspect de l'invention, on préfère transformer la bactérie *E. coli* selon l'invention par deux gènes exogènes provenant de bactéries différentes. On peut utiliser en particulier le gène d'invasion de la bactérie *Y. pseudotuberculosis* pour conférer la capacité d'entrer dans les cellules. On peut également utiliser le gène de l'hémolysine de *L. monocytogenes*, gène d'environ 2kb qui confère la capacité de dissémination intracellulaire en lysant les membranes des vacuoles.

Dans un mode de réalisation préféré, le système vectoriel selon l'invention est **caractérisé en ce que** ladite bactérie *E. coli* non pathogène est transformée par intégration chromosomique par le gène d'invasion de la bactérie ou *Y. pseudotuberculosis* qui confère la propriété de pénétrer dans le cytoplasme des cellules épithéliales.

Dans un mode de réalisation préféré, le système vectoriel selon l'invention est **caractérisé en ce que** ladite bactérie *E. coli* non pathogène est transformée par intégration chromosomique par le gène codant pour l'hémolysine de *L. monocytogenes* ou d'une autre *E. coli* pathogène qui confère la propriété de lyser les membranes des vacuoles desdites cellules cibles.

La fonction de reconnaissance et de pénétration de la membrane cellulaire et la fonction de lyse des membranes des vacuoles ou phagosomes, cette dernière permettant d'atteindre le cytoplasme, peuvent être conférées par un ou plusieurs gènes distincts notamment deux gènes distincts.

Dans un mode de réalisation préféré, le système vectoriel selon l'invention est **caractérisé en ce que** ladite bactérie *E. coli* non pathogène est transformée par intégration chromosomique à la fois par le gène d'invasion de la bactérie *Y. pseudotuberculosis* qui confère la propriété de pénétrer dans le cytoplasme des cellules épithéliales. Et par le gène codant pour l'hémolysine de *L. monocytogenes* qui confère la propriété de lyser les membranes des vacuoles desdites cellules cibles.

Dans un mode de réalisation préféré, le système vectoriel selon l'invention est **caractérisé en ce que** ladite bactérie *E. coli* non pathogène est transformée par intégration chromosomique à la fois par :
1. par le gène d'invasion *inv* de *Yersinia pseudotuberculosis,* de préférence de séquence SEQ ID NO: 10 ou de séquence identique à au moins 80 % et capable de conférer la propriété de pénétrer dans le cytoplasme des cellules épithéliales ;
2. le gène *hly* codant pour l'hémolysine de *Listeria monocytogenes*, de préférence de séquence SEQ ID NO: 4 ou de séquence identique à au moins 80 % et capable de conférer la propriété de lyser les membranes des vacuoles.

Dans un mode de réalisation préféré, le système vectoriel selon l'invention est caractérisé en ce que :
1. le gène d'invasion *inv* de *Yersinia pseudotuberculosis* est sous le contrôle du promoteur *Ptrc* ; et/ou
2. le gène *hly* codant pour l'hémolysine de *Listeria monocytogenes* est sous le contrôle du promoteur P*tet*.

Dans un mode de réalisation préféré, le système vectoriel selon l'invention est caractérisé en ce que ladite souche *E. coli* a été rendue incapable de survivre dans lesdites cellules dès son entrée dans le cytoplasme de cellules eucaryotes.

La souche de *E. coli* peut être rendue incapable de se multiplier et de survivre dans les cellules eucaryotes de multiples façons, en particulier en la rendant auxotrophe pour un facteur nécessaire à sa survie et absent des cellules eucaryotes.

Dans le mode de réalisation préféré selon l'invention, la bactérie *E. coli* est rendue incapable de se multiplier et donc de survivre dès son entrée dans le cytoplasme des cellules eucaryotes. Pour ce faire, dans un mode de réalisation préféré selon l'invention, ladite bactérie *E. coli* a été modifiée de manière a être rendue auxotrophe pour l'acide diaminopimélique qui est un composé essentiel de la synthèse de la paroi bactérienne et dont la voie de biosynthèse est bien connue.

Dans un mode de réalisation encore préféré, la bactérie *E. coli* est rendue dap⁻ à la suite d'un double événement de recombinaison homologue dans deux gènes de synthèse de l'acide diaminopimélique qui est spécifique des procaryotes et absent dans le cyptoplasme des cellules eucaryotes.

Les deux premières étapes de la synthèse du diaminopimélate qui n'est pas présent dans les cellules de mammifères, sont catalysées par les enzymes codées par les gènes *dapA* et *dapB*.

Le gène peut être doublement inactivé par délétion et insertion-inactivation dans les gènes *dapA* et *dapB*.

L'inactivation de la chaîne métabolique au niveau de la première étape évite l'accumulation dans les cellules d'un intermédiaire métabolique qui peut être métabolisé par une autre enzyme de la cellule.

Dans un mode de réalisation préféré, le système vectoriel selon l'invention est **caractérisé en ce que** l'un des gènes conférant à ladite bactérie *E. coli* recombinante non pathogène la capacité de pénétrer dans le cytoplasme desdites cellules cibles eucaryotes est intégré dans le chromosome de ladite *E. coli* par recombinaison homologue au niveau du gène *dapA* ou *dapB* de ladite bactérie *E. coli* non pathogène, aboutissant à une délétion au moins partielle de ce gène et à son inactivation.

Dans un mode de réalisation préféré, le système vectoriel selon l'invention est **caractérisé en ce que** l'un des gènes ou les deux gènes conférant à ladite bactérie *E. coli* recombinante non pathogène la capacité de pénétrer dans le cytoplasme desdites cellules cibles eucaryotes sont intégrés dans le chromosome de ladite *E. coli* par :
- insertion du gène de pénétration et délétion du gène *dapA* entre son fragment 5' de séquence SEQ ID NO: 3 et son fragment 3' de séquence SEQ ID NO: 5, ou entre un fragment 5' et 3' du gène *dapA* résultant en la délétion d'un fragment du gène *dapA* suffisant pour inactiver ce gène et/ou rendre auxotrophe ladite bactérie *E. coli* non pathogène pour l'acide diaminopimélique ;
- insertion du gène de lyse et délétion du gène *dapB* entre son fragment 5' de séquence SEQ ID NO: 9 et son fragment 3' de séquence SEQ ID NO: 11, ou entre un fragment 5' et 3' du gène *dapB* résultant en la délétion d'un fragment du gène *dapB* suffisant pour inactiver ce gène et/ou rendre auxotrophe ladite bactérie *E. coli* non pathogène pour l'acide diaminopimélique.

Dans un mode de réalisation préféré, le système vectoriel selon l'invention est **caractérisé en ce que** la sélection des bactéries *E. coli* recombinantes non pathogènes ayant intégré le gène de pénétration est effectuée au moyen d'une cassette de résistance à la tétracycline flanquée de sites FRT permettant son excision du chromosome bactérien par la recombinase Flp de levure, de préférence par l'introduction d'un plasmide dans ladite bactérie *E. coli* capable de produire la recombinase Flp sous forme transitoire.

Dans un mode de réalisation préféré, le système vectoriel selon l'invention est **caractérisé en ce que** l'intégration chromosomique du ou des gènes conférant à ladite bactérie *E. coli* recombinante non pathogène la capacité de pénétrer dans le cytoplasme desdites cellules cibles eucaryotes, est réalisée par recombinaison homologue en présence de protéines Red α(exo) et Red β (bet) du bactériophage λ exprimées par un plasmide.

Dans un mode de réalisation préféré, le système vectoriel selon l'invention est **caractérisé en ce qu**'il s'agit d'une bactérie *E. coli* recombinante non pathogène de génotype :
- [thi-1, endA1, hsdR17 (rK⁻ mκ⁺), supE44, Δ(lac)X74, ΔdapAΩinv, ΔdapBΩhly, recA1] ; ou
- [BM2710, ΔdapAΩinv, ΔdapBΩhly] ou [BM2710, ΔdapAΩPtrc-inv, ΔdapBΩPtet-hly], la souche BM2710 ayant été déposée le 27 octobre 1995 sous le numéro I-1635 à la CNCM ; ou
- la souche *E. coli* BM4570 déposée le 18 juillet 2007 sous le numéro I-3788 à la CNCM (Collection Nationale de Cultures de Microorganismes), Institut Pasteur, 25 Rue du Docteur Roux, 75724 Paris Cédex 15, France.

Un des problèmes lors de l'utilisation *in vivo* de *E. coli* est sa toxicité potentielle liée au LPS. Il a été montré récemment que l'on pouvait réduire le potentiel proinflammatoire du LPS en atténuant l'immunogénicité du lipide A par modification génétique (Somerville et al., 1996, d'Hauteville et al., 2002).

Ainsi sous un aspect particulier, la présente invention a pour objet un système vectoriel selon l'invention, **caractérisé en ce que** le gène de structure *msbB* de ladite bactérie *E. coli* recombinante non pathogène a été muté afin de générer une souche dont le lipide A du LPS est dépourvu d'acides gras myristoylés.

Dans un mode de réalisation particulièrement préféré, le système vectoriel selon l'invention est **caractérisé en ce qu**'il s'agit d'une bactérie *E. coli* recombinante non pathogène de génotype :
- [thi-1, endA1, hsdR17 (rκ⁻, mκ⁺), supE44, Δ(lac)X74, ΔmsbB, ΔdapAΩinv, ΔdapBΩhly, recA1] ; ou
- [BM2710, ΔmsbB, ΔdapAΩinv, ΔdapBΩhly] ou [BM2710, ΔmsbB,ΔdapAΩPtrc-inv, ΔdapBΩPtet-hly], la souche BM2710 ayant été déposée le 27 octobre 1995 sous le numéro I-1635 à la CNCM ; ou
- la souche *E. coli* BM4573 déposée le 18 juillet 2007 sous le numéro I-3790 à la CNCM (Collection Nationale de Cultures de Microorganismes), Institut Pasteur, 25 Rue du Docteur Roux, 75724 Paris Cédex 15, France.

La présente invention permet également la génération d'une souche de *E coli* invasive permettant la production en grande quantité par le vecteur de protéines hétérologues et de « short hairpin » RNA (sh RNA, molécules de RNA interférant synthétisés par la bactérie) sous contrôle du promoteur T7, le gène codant pour la T7 RNA polymérase est intégré dans le chromosome bactérien.

Ainsi, la présente invention comprend un procédé in vitro, ex vivo ou in vivo de production de protéines hétérologues ou d'acide nucléique, notamment des ARN, notamment des ARN de type « short hairpin RNA » (pour ARN court en épingle à cheveux), dans une cellule eucaryote, **caractérisé en ce qu'**il met en oeuvre un système vectoriel selon l'invention dans lequel ledit acide nucléique est sous le contrôle du promoteur T7 et le gène codant pour la T7 RNA polymérase est intégré dans le chromosome bactérien de ladite bactérie *E. coli* recombinante.

Dans un mode de réalisation préféré, le système vectoriel selon l'invention est **caractérisé en ce que** le gène de la T7 RNA polymérase a été intégré, de préférence sous le contrôle du promoteur *lacUV5*, dans le chromosome ladite bactérie *E. coli* recombinante non pathogène.

Dans un mode de réalisation particulièrement préféré, le système vectoriel selon l'invention est **caractérisé en ce qu**'il s'agit d'une bactérie *E. coli* recombinante non pathogène de génotype :
- [thi-1, endA1, hsdR17 (rκ⁻ mκ⁺), supE44, Δ(lac)X74, ΔmsbB, ΔdapAΩinv, ΔdapBΩhly, recA1, (DE3)] ; ou
- [BM2710, ΔdapAΩinv, ΔdapBΩhly, (DE3)] ou [BM2710, ΔdapAΩPtrc-inv, ΔdapBΩPtet-hly, (DE3)], la souche BM2710 ayant été déposée le 27 octobre 1995 sous le numéro I-1635 à la CNCM ; ou
- la souche *E. coli* BM4570 (DE3) déposée le 18 juillet 2007 sous le numéro 1- 3789 à la CNCM (Collection Nationale de Cultures de Microorganismes), Institut Pasteur, 25 Rue du Docteur Roux, 75724 Paris Cédex 15, France.

Dans un mode de réalisation préféré, le système vectoriel selon l'invention est **caractérisé en ce que** ladite souche *E coli* recombinante non pathogène est transformée par un vecteur réplicatif ou non dans *E coli* portant ledit acide nucléique d'intérêt ou codant pour ladite protéine d'intérêt, et, le cas échéant, placé sous le contrôle d'éléments de régulation dans lesdites cellules cibles eucaryotes.

Avantageusement, ledit acide nucléique d'intérêt comporte un fragment d'ADN codant pour une protéine d'intérêt, ce dernier fragment étant placé sous le contrôle d'éléments de régulation dans lesdites cellules cibles eucaryotes.

On entend par « éléments de régulation » les séquences régulatrices appropriées pour la transcription puis la traduction telles que promoteur, y compris codons « start » et « stop », « enhancer » et « opérateur ». Les moyens et méthodes pour identifier et sélectionner ces éléments sont bien connus de l'homme de l'art.

Dans un mode de réalisation avantageux, ladite souche est transformée par un vecteur réplicatif ou non réplicatif dans *E. coli* portant ledit acide nucléique d'intérêt et éventuellement lesdits éléments de régulation.

Dans un mode de réalisation préféré, le système vectoriel selon l'invention est **caractérisé en ce que** ledit vecteur portant ledit acide nucléique d'intérêt comporte en outre des éléments permettant l'intégration dans le génome des cellules eucaryotes cibles.

Dans un mode de réalisation préféré, le système vectoriel selon l'invention est **caractérisé en ce que** ledit vecteur portant ledit acide nucléique d'intérêt comporte en outre une origine de réplication permettant au vecteur de se répliquer de façon extra chromosomique dans lesdites cellules eucaryotes cibles.

Selon la présente invention, ledit acide nucléique d'intérêt est porté par un plasmide réplicatif et non intégratif dans la bactérie. En outre, le fragment d'ADN étranger est placé sous le contrôle d'éléments d'expression fonctionnelle dans les cellules eucaryotes cibles.

Ledit vecteur portant peut être réplicatif ou intégratif dans les cellules eucaryotes cibles, c'est-à-dire qu'il peut comporter des éléments permettant l'intégration dans le génome des cellules eucaryotes cibles, ou comporter une origine de réplication permettant au vecteur de se répliquer de façon extra chromosomique dans les cellules eucaryotes cibles.

Dans un mode de réalisation préféré, le système vectoriel selon l'invention est **caractérisé en ce que** lesdites cellules eucaryotes sont des cellules de mammifère, des cellules de levure ou de plante, de préférence des cellules de mammifère.

Sous un autre aspect, la présente invention a pour objet un système vectoriel selon l'invention pour la préparation d'une composition thérapeutique.

Sous un autre aspect, la présente invention a pour objet une méthode de transfert *in vivo* ou *in vitro* d'ADN dans des cellules eucaryotes autres que des cellules animales ou humaines, caractérisée en ce qu'elle met en oeuvre un système vectoriel selon l'invention.

Sous un autre aspect, la présente invention a pour objet une méthode *in vitro* ou *ex vivo* de transfert d'ADN dans des cellules eucaryotes humaines ou animales à partir d'un échantillon biologique d'origine humaine ou animale, caractérisée en ce qu'elle met en oeuvre un système vectoriel selon l'invention.

Sous un autre aspect, la présente invention a pour objet l'utilisation d'un système vectoriel selon l'invention, pour la préparation d'une composition vaccinale, **caractérisée en ce que** l'acide nucléique d'intérêt code pour un antigène d'un agent infectieux dont l'infection peut être prévenue au moyen d'un anticorps dirigé contre cet antigène.

Sous un autre aspect, la présente invention a pour objet l'utilisation d'un système vectoriel selon l'invention, la préparation d'une composition vaccinale anti-tumorale, **caractérisée en ce que** l'acide nucléique d'intérêt code pour un antigène tumoral dont la tumeur ou le cancer peut être prévenu au moyen d'un anticorps dirigé contre cet antigène.

Sous un dernier aspect, la présente invention a pour objet l'utilisation d'un système vectoriel selon l'invention, pour la préparation d'une composition thérapeutique destinée au traitement ou à la prévention de maladies par thérapie génique.

D'autres caractéristiques et avantages de la présente invention apparaîtront à la lumière des exemples et des figures ci-après.

### Légendes des figures

Figure 1 : la figure 1 représente un schéma de construction des vecteurs bactériens.
Figure 2 : la figure 2 représente un schéma montrant les différentes étapes de constructions pour aboutir aux différents systèmes vectoriels BM4570, BM4573 et BM4570(DE3).
Figures 3A-3C : les figures 3A à 3C représentent les résultats comparatifs obtenus par analyse FACS pour les différents systèmes vectoriels dérivés de la souche BM2710, expression du gène *inv* à la surface de la bactérie *E. coli.*
Figure 4 : la figure 4 représente la quantification de l'activité hémolityque dans les trois vecteurs.
Figure 5 : la figure 5 représente la capacité de transfert du gène pEGFP-C1 par BM4570 par comparaison avec celle de BM2710pGB2Ω*inv-hly*.

### EXEMPLE 1 : Construction de E. coli BM4570 par intégration des gènes inv et hly dans le chromosome de E. coli BM2710

Il a été montré que l'expression des protéines Red α(exo) et Red β(bet) du bactériophage λ, ainsi que la protéine Red γ (gam) pouvait promouvoir de façon efficace la recombinaison homologue chez une souche de *E. coli recA* dépourvue de recombinaison homologue. En utilisant ce système il est donc possible d'effectuer l'échange allélique de gènes situés dans le chromosome à la suite d'un double évènement de recombinaison en électroporant dans la souche à modifier un fragment d'ADN contenant des séquences terminales homologues à la cible (J.P.P. Muyrers, 2001). Les fonctions Red αβγ du phage λ sont portées par le plasmide pKOBEGA qui possède une origine de réplication thermosensible et sont exprimées sous le contrôle du promoteur inductible par l'arabinose pBAD (Chaveroche et al., 2000).

La souche de *E. coli* hébergeant pKOBEGA a été électrotransformée par des fragments qui incluent le gène *inv* ou le gène *hly* flanqué, respectivement, par les portions 5' et 3' du gène *dapA* ou du gène *dapB*.

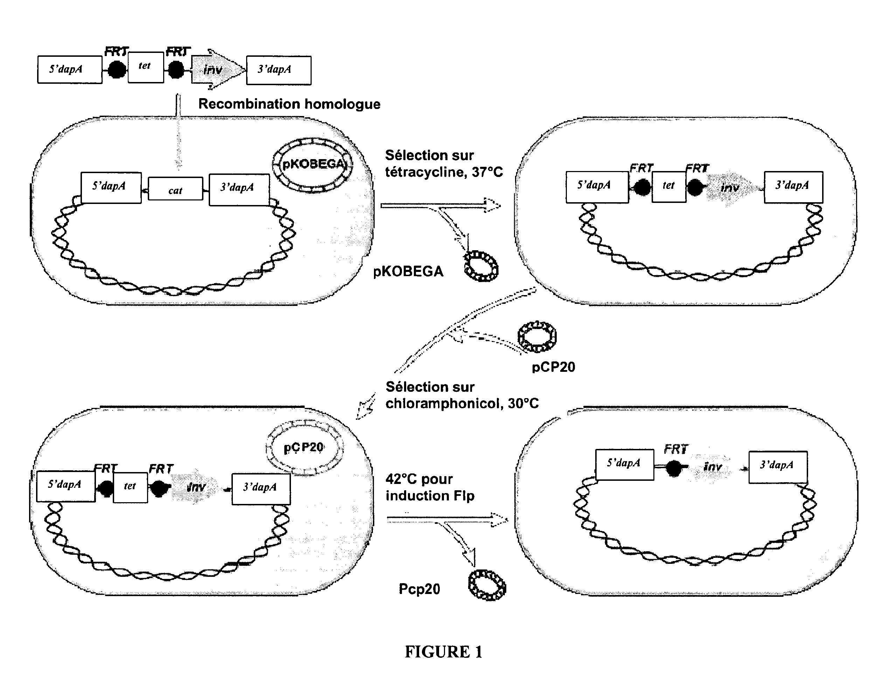

Pour sélectionner les souches recombinantes, nous avons utilisé une cassette de résistance à la tétracycline qu'il a été possible d'exciser secondairement du chromosome. La cassette tet^{R} (D. Mazel, non publié) est flanquée par de courtes répétitions directes (sites FRT) qui permettent son excision par la recombinase Flp de levure (M.M. Cox, 1983) générant ainsi un vecteur bactérien stable et dépourvu de caractère de résistance. La résistance à la tétracycline a été délétée du chromosome en introduisant dans la souche le plasmide pCP20 (Cherepanov et Wackernagel, 1995) qui permet la production transitoire de la recombinase Flp en *trans* (figure 1).

Nous avons intégré avec ce système :
- dans le gène *dapA* de *E. coli* BM2710, le gène *inv* sous le contrôle du promoteur fort P*trc*. P*trc* est un promoteur hybride composé de la séquence -35 du promoteur *trp* et de la séquence -10 de *lac*UV5 ;
- dans le gène *dapB* de *E. coli* BM2710, le gène *hly* dépourvu de séquence signal sous le contrôle du promoteur P*tet* de pACYC184 (Higgins et al., 1999).

### EXEMPLE 2 : Construction de E. coli BM4573 dérivé de E. coli BM2710 invasive avec LPS modifié

Des mutations dans le gène de structure *msbB* de *E. coli* générant des souches dont le lipide A du LPS est dépourvu d'acides gras myristoylés ce qui diminue ainsi leur activité proinflammatoire (Somerville et al., 1996).

Le plasmide pCVD442 *msbBl::km*, vecteur suicide qui ne réplique que dans des souches produisant la protéine Pir, a été utilisé (d'Hauteville et al., 2002). L'insertion dans ce plasmide comprend la partie 5' et la région en amont du gène *msbB*, le déterminant de résistance à la kanamycine *αphA* de pUC4K et la partie 3' et la région en aval du gène *msbB*. Le gène *αphA* de cette construction a été remplacé par la cassette *tet*^{R} flanquée des sites FRT. La souche de *E. coli* BM4573 (*ΔmsbB*) a été construite en utilisant la même approche que celle précédemment décrite.

### EXEMPLE 3 : Construction de E. coli BM4570 (DE3) par intégration du gène de la T7 RNA polymérase dans le chromosome de E. coli BM4570

Le λDE3 lysogenization kit (Novagen) a été utilisé pour intégrer le gène de la T7 RNA polymérase sous le contrôle du promoteur *lacUV5*.

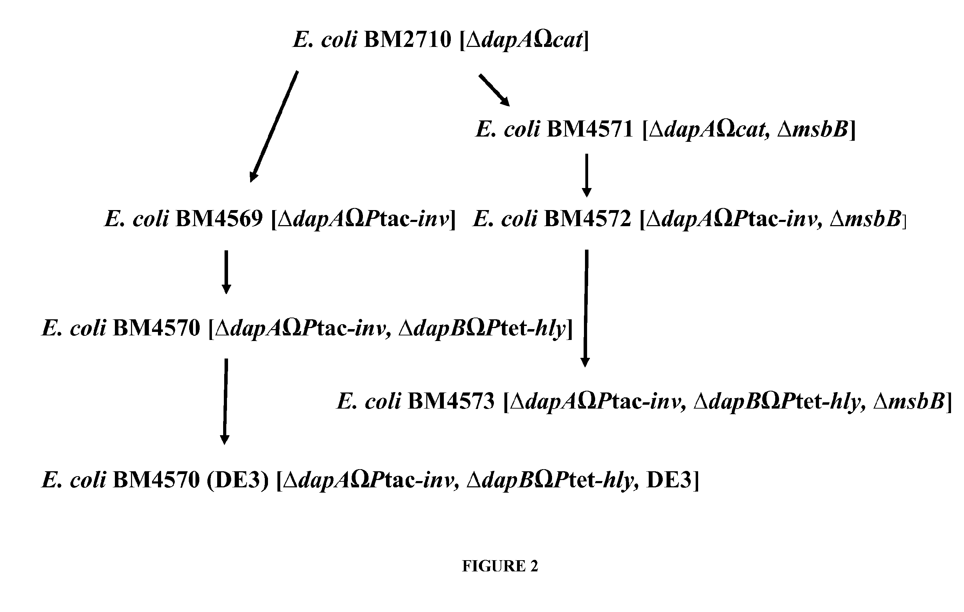

Les principales étapes de la construction des souches sont représentées dans la figure 2.

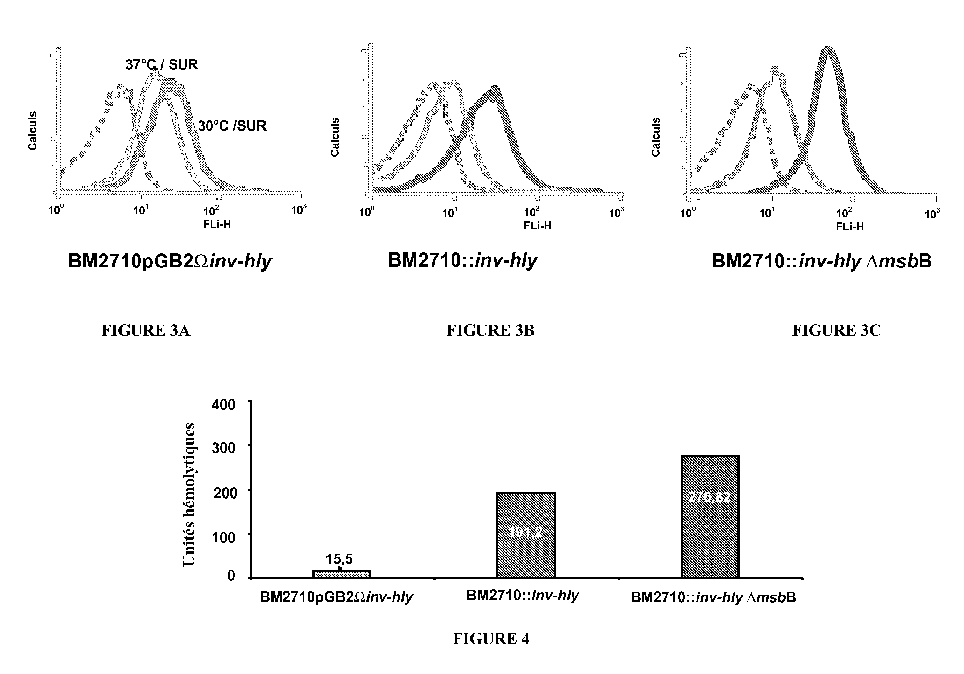

Pour chacune des souches construites, l'expression de l'invasine à la surface de la bactérie et la production de listériolysine ont été étudiées et comparées à celles de la souche originale qui héberge le plasmide pGB2Q*inv-hly* (figure 3).

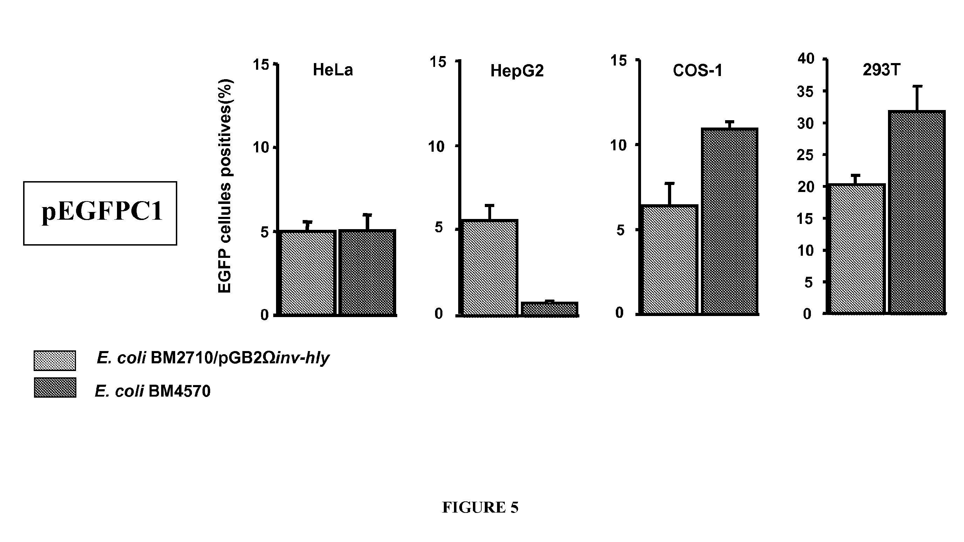

De même, la capacité de ces souches à transférer le gène EGFP a été étudiée de façon comparative (figure5).

### REFERENCES BIBLIOGRAPHIQUES

- Chaveroche MK, Ghigo JM, and d'Enfert C (2000) A rapid method for efficient gene replacement in the filamentous fungus Aspergillus nidulans. Nucleic Acids Res., 28(22) e97.
- Courvalin P, Goussard S and Grillot-Courvalin C (1995) Gene transfer from bacteria to mammalian cells. C.R. Acad. Sci. Ser.III., 318:1207-1212.
- Cox MM. (1983) The FLP protein of the yeast 2-µm plasmid: Expression of a eukaryotic genetic recombination system in Escherichia coli. Proc. Natl. Acad. Sci. USA, 80:4223-4227.
- d'Hauteville H, Khan S, Maskell DJ, Kussak A, Weintraub A, Mathison J, Ulevitch RJ, Wuscher N, Parsot C, and Sansonetti PJ (2002) Two msbB genes encoding maximal acylation of lipid A are required for invasive Shigella flexneri to mediate inflammatory rupture and destruction of the intestinal epithelium. J. Immunol., 168:5240-5251.
- Grillot-Courvalin C, Goussard S, Huetz F, Ojcius DM, and Courvalin P (1998) Functional gene transfer from intracellular bacteria to mammalian cells. Nature Biotechnol., 16:862-866.
- Robbens J, Raeymaekers A, Steidler L, Fiers W, and Remaut E (1995) Production of soluble and active recombinant murine interleukin-2 in Escherichia coli: high level expression, Kil-induced release, and purification. Protein Expr. Purif., 6:481-486.
- Somerville Jr. JE, Cassiano L, Bainbridge B, Cunningham MD, and Darveau RP (1996) A novel Escherichia coli lipid A mutant that produces an antiinflammatory lipopolysaccharide. J. Clin. Invest., 97(2):359-365.

## Revendications

1. Système vectoriel capable de délivrer dans des cellules cibles eucaryotes un acide nucléique d'intérêt ou codant pour une protéine d'intérêt, ce système vectoriel comprenant une bactérie *E. coli* recombinante non pathogène, ladite bactérie *E. coli* non pathogène étant en outre :
- modifiée par introduction (par échange allélique) d'un ou plusieurs gènes conférant à cette bactérie *E. coli* recombinante non pathogène la capacité de pénétrer dans le cytoplasme desdites cellules cibles eucaryotes ;
- incapable de survivre dans le cytoplasme de ladite cellule cible ; et
- modifiée par les fragments d'ADN étrangers,
**caractérisé en ce que** le ou les gènes conférant à ladite bactérie *E. coli* recombinante non pathogène la capacité de pénétrer dans le cytoplasme desdites cellules cibles eucaryotes sont intégrés dans le chromosome de ladite *E. coli* non pathogène.

2. Système vectoriel selon la revendication 1, **caractérisé en ce que** ladite bactérie *E. coli* non pathogène est transformée par intégration chromosomique de deux gènes, provenant d'une ou de deux autres bactéries, lui conférant la capacité de pénétrer dans le cytoplasme desdites cellules cibles.

3. Système vectoriel selon la revendication 1 ou 2, **caractérisé en ce que** le ou les gènes intégrés dans le chromosome de la bactérie *E. coli* non pathogène et lui conférant sa capacité de pénétrer dans le cytoplasme desdites cellules cibles, lui permettent de lyser la membrane des vacuoles desdites cellules cibles pour atteindre la cytoplasme.

4. Système vectoriel selon l'une des revendications 1 à 3, **caractérisé en ce que** ladite bactérie *E. coli* non pathogène est transformée par intégration chromosomique par le gène d'invasion de la bactérie *Y. pseudotuberculosis* qui confère la propriété de pénétrer dans le cytoplasme des cellules épithéliales.

5. Système vectoriel selon l'une des revendications 1 à 3, **caractérisé en ce que** ladite bactérie *E. coli* non pathogène est transformée par intégration chromosomique par le gène codant pour l'hémolysine de *L. monocytogenes* qui confère la propriété de lyser les membranes des vacuoles desdites cellules cibles.

6. Système vectoriel selon l'une des revendications 1 à 5, **caractérisé en ce que** ladite bactérie *E. coli* non pathogène est transformée par intégration chromosomique à la fois par le gène d'invasion de la bactérie *Y. pseudotuberculosis* qui confère la propriété de pénétrer dans le cytoplasme des cellules épithéliales et par le gène codant pour l'hémolysine de *L. monocytogenes* qui confère la propriété de lyser les membranes des vacuoles desdites cellules cibles et par le gène.

7. Système vectoriel selon l'une des revendications 1 à 6, **caractérisé en ce que** ladite bactérie *E. coli* non pathogène est transformée par intégration chromosomique à la fois par :
1. par le gène d'invasion *inv* de *Y. pseudotuberculosis,* de préférence de séquence SEQ ID NO: 10 ou de séquence identique à au moins 80 % et capable de conférer la propriété de pénétrer dans le cytoplasme des cellules épithéliales; et
2. le gène *hly* codant pour l'hémolysine de *L. monocytogenes,* de préférence de séquence SEQ ID NO: 4 ou de séquence identique à au moins 80 % et capable de conférer la propriété de lyser les membranes des vacuoles.

8. Système vectoriel selon la revendication 7, **caractérisé en ce que :**
1. le gène d'invasion *inv* de Yersinia pseudotuberculosis est sous le contrôle du promoteur *Ptet* ;
2. le gène *hly* codant pour l'hémolysine de *L. monocytogenes* est sous le contrôle du promoteur P*trc*.

9. Système vectoriel selon l'une des revendications 1 à 8, **caractérisé en ce que** ladite souche *E. coli* a été rendue incapable de survie dans lesdites cellules dès son entrée dans le cytoplasme de cellules eucaryotes.

10. Système vectoriel selon la revendication 9, **caractérisé en ce que** ladite bactérie *E. coli* recombinante non pathogène a été modifiée de manière à être rendue auxotrophe pour l'acide diaminopimélique.

11. Système vectoriel selon la revendication 10, **caractérisé en ce que** le gène conférant à ladite bactérie *E. coli* recombinante non pathogène la capacité de pénétrer dans le cytoplasme desdites cellules cibles eucaryotes est intégré dans le chromosome de ladite bactérie *E. coli* par recombinaison homologue au niveau du gène *dapA* de ladite bactérie *E. coli* non pathogène, aboutissant à une délétion au moins partielle de ce gène et à son inactivation, de préférence que le gène *dapA* codant pour l'enzyme dihydrodipicolinate synthase est inactivée.

12. Système vectoriel selon la revendication 10 ou 11, **caractérisé en ce que** le gène conférant à ladite bactérie *E. coli* recombinante non pathogène la capacité de pénétrer dans le cytoplasme desdites cellules cibles eucaryotes est intégré dans le chromosome de ladite bactérie *E. coli* par recombinaison homologue au niveau du gène *dapB* de ladite bactérie *E. coli* non pathogène, aboutissant à une délétion au moins partielle de ce gène et à son inactivation.

13. Système vectoriel selon l'une des revendications 9 à 12, **caractérisé en ce que** le gène conférant à ladite bactérie *E. coli* recombinante non pathogène la capacité de pénétrer dans le cytoplasme desdites cellules cibles eucaryotes et de lyser la vacuole de pénétration est intégré dans le chromosome de ladite bactérie *E. coli* par :
- insertion du gène de pénétration et délétion du gène *dapA* entre son fragment 5' de séquence SEQ ID NO: 3 et son fragment 3' de séquence SEQ ID NO: 5, ou entre un fragment 5' et 3' du gène *dapA* résultant en la délétion d'un fragment du gène *dapA* suffisant pour inactiver ce gène et/ou rendre auxotrophe ladite bactérie *E. coli* non pathogène pour l'acide diaminopimélique ;
- insertion du gène de lyse et délétion du gène *dapB* entre son fragment 5' de séquence SEQ ID NO: 9 et son fragment 3' de séquence SEQ ID NO: 11, ou entre un fragment 5' et 3' du gène *dapB* résultant en la délétion d'un fragment du gène *dapB* suffisant pour inactiver ce gène et/ou rendre auxotrophe ladite bactérie *E. coli* non pathogène pour l'acide diaminopimélique.

14. Système vectoriel selon l'une des revendications 9 à 13, **caractérisé en ce que** la sélection des bactéries *E. coli* recombinantes non pathogènes ayant intégré le gène de pénétration et le gène de lyse est effectuée au moyen d'une cassette de résistance à la tétracycline flanquée de sites FRT permettant son excision du chromosome bactérien par la recombinase Flp de levure, de préférence par l'introduction d'un plasmide dans ladite bactérie *E. coli* capable de produire la recombinase Flp sous forme transitoire.

15. Système vectoriel selon l'une des revendications 1 à 14, **caractérisé en ce que** l'intégration chromosomique du ou des gènes conférant à ladite bactérie *E. coli* recombinante non pathogène la capacité de pénétrer dans le cytoplasme et de lyser la vacuole de pénétration desdites cellules cibles eucaryotes, est réalisée par recombinaison homologue en présence de protéines Red α(exo) et Red β (bet) du bactériophage λ exprimées par un plasmide.

16. Système vectoriel selon l'une des revendications 1 à 15, **caractérisé en ce qu'**il s'agit d'une bactérie *E. coli* recombinante non pathogène de génotype :
- [thi-1, endA1, hsdR17 (rκ⁻ mκ⁺), supE44, Δ(lac)X74, ΔdapAΩinv, ΔdapBΩhly, recA1] ; ou
- [BM2710, ΔdapAΩinv, ΔdapBΩhly] ou [BM2710, ΔdapAΩPtrc-inv, ΔdapBΩPtet-hly], la souche BM2710 ayant été déposée le 27 octobre 1995 sous le numéro I-1635 à la CNCM de génotype ; ou
- la souche *E. coli* BM4570 déposée le 18 juillet 2007 sous le numéro I-3788 à la CNCM (Collection Nationale de Cultures de Microorganismes), Institut Pasteur, 25 Rue du Docteur Roux, 75724 Paris Cédex 15, France.

17. Système vectoriel selon l'une des revendications 1 à 16, **caractérisé en ce que** le gène de structure *msbB* de ladite bactérie *E. coli* recombinante non pathogène a été muté afin de générer une souche dont le lipide A du LPS est dépourvu d'acides gras myristoylés.

18. Système vectoriel selon la revendication 17, **caractérisé en ce qu'**il s'agit d'une bactérie *E. coli* recombinante non pathogène de génotype :
- [thi-1, endA1, hsdR17 (rκ⁻, mκ⁺), supE44, Δ(lac)X74, ΔmsbB, ΔdapAΩinv, ΔdapBΩhly, recA1] ; ou
- [BM2710, ΔmsbB, ΔdapAΩinv, ΔdapBΩhly] ou [BM2710, ΔmsbB,ΔdapAΩPtrc-inv, ΔdapBΩPtet-hly], la souche BM2710 ayant été déposée le 27 octobre 1995 sous le numéro I-1635 à la CNCM de génotype ; ou
- la souche *E. coli* BM4573 déposée le 18 juillet 2007 sous le numéro I-3790 à la CNCM (Collection Nationale de Cultures de Microorganismes), Institut Pasteur, 25 Rue du Docteur Roux, 75724 Paris Cédex 15, France.

19. Système vectoriel selon lune des revendications 1 à 18, **caractérisé en ce que** ledit acide nucléique d'intérêt est de toutes tailles jusqu'à 100 kb ou de 100 à 150 kb ou plus grand que 150 kb.

20. Système vectoriel selon l'une des revendications 1 à 18, **caractérisé en ce que** l'acide nucléique d'intérêt, de préférence codant pour une protéine hétérologue ou un ARN de type shRNA, est sous le contrôle du promoteur T7, le gène codant pour la T7 RNA polymérase étant intégré dans le chromosome bactérien.

21. Système vectoriel selon l'une des revendications 1 à 18, **caractérisé en ce que** le gène de la T7 RNA polymérase a été intégré, de préférence sous le contrôle du promoteur *lacUV5*, dans le chromosome de ladite bactérie *E. coli* recombinante non pathogène.

22. Système vectoriel selon la revendication 21, **caractérisé en ce qu'**il s'agit d'une bactérie *E. coli* recombinante non pathogène de génotype :
- [thi-1, endA1, hsdR17 (rκ⁻ mκ⁺), supE44, Δ(lac)X74, ΔmsbB, ΔdapAΩinv, ΔdapBΩhly, recA1, (DE3)] ; ou
- [BM2710, ΔdapAΩinv, ΔdapBΩhly, (DE3)] ou [BM2710, ΔdapAΩPtrc-inv, ΔdapBΩPtet-hly, (DE3)], la souche BM2710 ayant été déposée le 27 octobre 1995 sous le numéro I-1635 à la CNCM de génotype ; ou
- la souche *E. coli* BM4570 (DE3) déposée le 18 juillet 2007 sous le numéro I-3789 à la CNCM (Collection Nationale de Cultures de Microorganismes), Institut Pasteur, 25 Rue du Docteur Roux, 75724 Paris Cédex 15, France.

23. Système vectoriel selon lune des revendications 1 à 22, **caractérisé en ce que** ladite souche *E. coli* recombinante non pathogène est transformée par un vecteur réplicatif ou non dans *E. coli* portant ledit acide nucléique d'intérêt ou codant pour ladite protéine d'intérêt, et, le cas échéant, placé sous le contrôle d'éléments de régulation dans lesdites cellules cibles eucaryotes.

24. Système vectoriel selon la revendication 22 ou 23, **caractérisé en ce que** ledit vecteur portant ledit acide nucléique d'intérêt comporte en outre des éléments permettant l'intégration dans le génome des cellules eucaryotes cibles.

25. Système vectoriel selon la revendication 22 ou 23, **caractérisé en ce que** ledit vecteur portant ledit acide nucléique d'intérêt comporte en outre une origine de réplication permettant au vecteur de se répliquer de façon extra chromosomique dans lesdites cellules eucaryotes cibles.

26. Système vectoriel selon l'une des revendications 1 à 25, **caractérisé en ce que** lesdites cellules eucaryotes sont des cellules de mammifère, des cellules de levure ou de plante, de préférence des cellules de mammifère.

27. Système vectoriel selon l'une des revendications 1 à 26, pour la préparation d'une composition thérapeutique.

28. Méthode de transfert *in vivo* ou *in vitro* d'ADN dans des cellules eucaryotes autres que des cellules animales ou humaines, **caractérisée en ce qu'**elle met en oeuvre un système vectoriel selon l'une des revendications 1 à 26.

29. Méthode *in vitro* ou *ex vivo* de transfert d'ADN et d'ARN dans des cellules eucaryotes humaines ou animales à partir d'un échantillon biologique d'origine humaine ou animale, **caractérisée en ce qu'**elle met en oeuvre un système vectoriel selon l'une des revendications 1 à 26.

30. Utilisation d'un système vectoriel selon l'une des revendications 1 à 27, pour la préparation d'une composition vaccinale, **caractérisée en ce que** l'acide nucléique d'intérêt code pour un ou plusieurs antigènes d'un agent infectieux ou pour des fragments antigéniques.

31. Utilisation d'un système vectoriel selon l'une des revendications 1 à 27, pour la préparation d'une composition vaccinale anti-tumorale, **caractérisée en ce que** l'acide nucléique d'intérêt code pour un antigène tumoral.

32. Utilisation d'un système vectoriel selon l'une des revendications 1 à 27, pour la préparation d'une composition thérapeutique destinée au traitement ou à la prévention de maladies par thérapie génique.
